# FASCICULE DE BREVET EUROPEEN

(11) **EP 0 874 812 B1**
(45) Date de publication et mention de la délivrance du brevet: **05.04.2000**
(21) Numéro de dépôt: 96939987.2
(22) Date de dépôt: 27.11.1996
(51) Int. Cl.: C07C 323/60, C07C 317/44, C07C 235/24, A61K 31/16

(54) **NOUVEAUX DERIVES DE 2,3,5 TRIMETHYL-4-HYDROXY ANILIDES, LEUR PREPARATION ET LEUR APPLICATION EN THERAPEUTIQUE**
2,3,5-TRIMETHYL-HYDROXYANILID-DERIVATIVE, IHRE HERSTELLUNG UND IHRE THERAOEUTISCHE VERWENDUNG
NOVEL 2,3,5-TRIMETHYL-4-HYDROXY ANILIDE DERIVATIVES, PREPARATION THEREOF AND THERAPEUTICAL USE THEREOF

(30) Priorité: 28.11.1995 FR 9514086
(43) Date de publication de la demande: 04.11.1998
(73) Titulaire: PIERRE FABRE MEDICAMENT, 92100 Boulogne (FR)
(72) Inventeur: PATOISEAU, Jean-François, F-81100 Castres (FR); AUTIN, Jean-Marie, F-81290 Labruguière (FR); DELHON, André, F-81100 Castres (FR); OMS, Philippe, F-81100 Castres (FR); JUNQUERO, Didier, F-81090 Burlats (FR)
(74) Mandataire: Ahner, Francis
(86) Numéro de dépôt international: FR9601877
(87) Numéro de publication internationale: WO9719918

(56) Documents cités:
- EP-A- 0 559 898
- EP-A- 0 619 312
- PATENT ABSTRACTS OF JAPAN vol. 18, no. 262 (C-1201), 19 Mai 1994 & JP 06 040898 A (TAISHO PHARMACEUTICAL), 15 Février 1994,

## Description

La présente invention a pour objet de nouveaux dérivés d'anilides, leur préparation et leur application en thérapeutique humaine.

Elle concerne également l'utilisation de ces dérivés pour la fabrication de médicaments destinés au traitement de l'hypercholestérolémie ou de l'athérosclérose.

Le cholestérol alimentaire est absorbé sous forme de cholestérol libre par les cellules intestinales, puis estérifié par l'enzyme ACAT (acyl CoA : cholestérol O - Acyl transférase), dans le sérum. L'inhibition de l'ACAT prévient l'absorption intestinale et l'accumulation de cholestérol dans le tissu artériel. En outre, les lipoprotéines de basse densité (LDL) sont, après oxydation, captées par les "Scavenger receptors" et conduisent à la formation de la cellule spumeuse, point de départ de la plaque d'athérome. (D. STEINBERG et al., New England. J. Med. 320, 915-924, 1989).

EP-A-559 898 et JP 60 40 898 décrivent des dérivés N-(2,6-diisopropylphényl)-2-(alkylsulfonyl)-acétamide ainsi que des dérivés N-(2,6-diisopropylphényl)-2-(alkylthio)-acétamide ou propanamide comme inhibiteurs de l'enzyme ACAT. Ces dérivés sont obtenus par action d'un thiol sur le N-(2,6-diisopropylphényl)-2-chloro-acétamide ou propanamide, en présence d'une base.

EP-A-619 312 décrit également des dérivés anilides inhibiteurs de l'enzyme ACAT du type N-(2,6-diisopropylphényl)-2-[6-(4-pipérazinyl)hexylthio]acétamide.

L'objet de la présente invention vise à obtenir de nouveaux dérivés hypocholestérolémiants et anti-oxydants pouvant agir à la fois sur la quantité et la qualité des L.D.L dans le but de réduire leur potentiel athérogène et leurs effets délétères à long terme sur la paroi vasculaire.

Les composés de la présente invention répondent à la formule générale I. 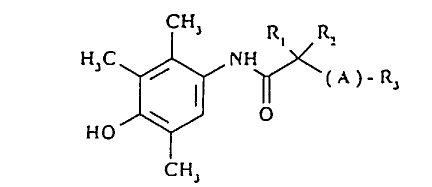 dans laquelle :
R₁ et R₂ identiques ou différents représentent indépendamment l'un de l'autre :
- l'hydrogène
- un radical alcoyle linéaire ou ramifié en C₁ - C₆
- un groupement aromatique tel que phényle, naphtyle ou pyridyle éventuellement substitué par un ou plusieurs groupements alcoyle en C₁ - C₄, alcoxy en C₁ - C₄, hydroxyle ou halogéno,

. R3 représente une chaîne alcoyle linéaire ou ramifiée en C₆ - C₁₅ ou un groupement phényle éventuellement substitué par un ou plusieurs groupements alcoyle en C₁ - C₄ alcoxy en C₁ - C₄, hydroxyle ou halogéno,
. A représente un atome d'oxygène ou de soufre ou le groupement sulfoxy.

Les composés de formule générale 1 pouvant posséder des centres asymétriques, la présente invention couvre les différents stéréoisomères ou énantiomères et leurs mélanges.

Les composés de formule générale I peuvent être utilisés pour la préparation de compositions pharmaceutiques ou de médicaments destinés au traitement de maladies telles que l'hypercholestérolémie ou l'athérosclérose.

Enfin, les procédés de synthèse permettant d'accéder aux composés de formule générale I font également partie de la présente invention.

Les composés de formule générale I peuvent être obtenus selon l'une des méthodes suivantes (schéma I) :

### Méthode A :

a) Traitement du chlorhydrate de 2,3,6-triméthyl-4-amino phénol par un α halogéno halogénure d'acyle II, dans lequel Hal et Hal' représentent le brome ou le chlore et R₁, R₂ ont la même signification que précédemment en présence d'une base telle que la triéthylamine, pour accéder au composé III.
b) Traitement du composé III par le dérivé IV, dans lequel R₃ et A ont la même signification que précédemment, dans un milieu sodium - méthanol ou tertiobutylate de potassium - tertiobutanol, pour donner le composé I.

### Méthode B :

- Traitement du chlorhydrate 2,3,6-triméthyl-4-amino phénol par un α halogéno acide V, dans lequel Hal, R₁, R₂ ont la même signification que précédemment, en présence d'un activateur tel que le dicyclohexyl carbodiimide ou l'iodure de 2-chloro-1-méthyl-pyridinium et d'une base telle que la triéthylamine, pour accéder au composé III, traité ensuite de façon identique à celle décrite dans la méthode A-b.

### Méthode C :

- Traitement du chlorhydrate 2,3,6-triméthyl-4-amino phénol par le dérivé IV, dans lequel R₁,R₂,R₃ et A ont la même signification que précédemment, soit en présence d'un activateur tel que le dicyclo hexyl carbodiimide ou l'iodure de 2-chloro-1-méthyl pyridinium, et de triéthyl amine, pour donner le composé I.

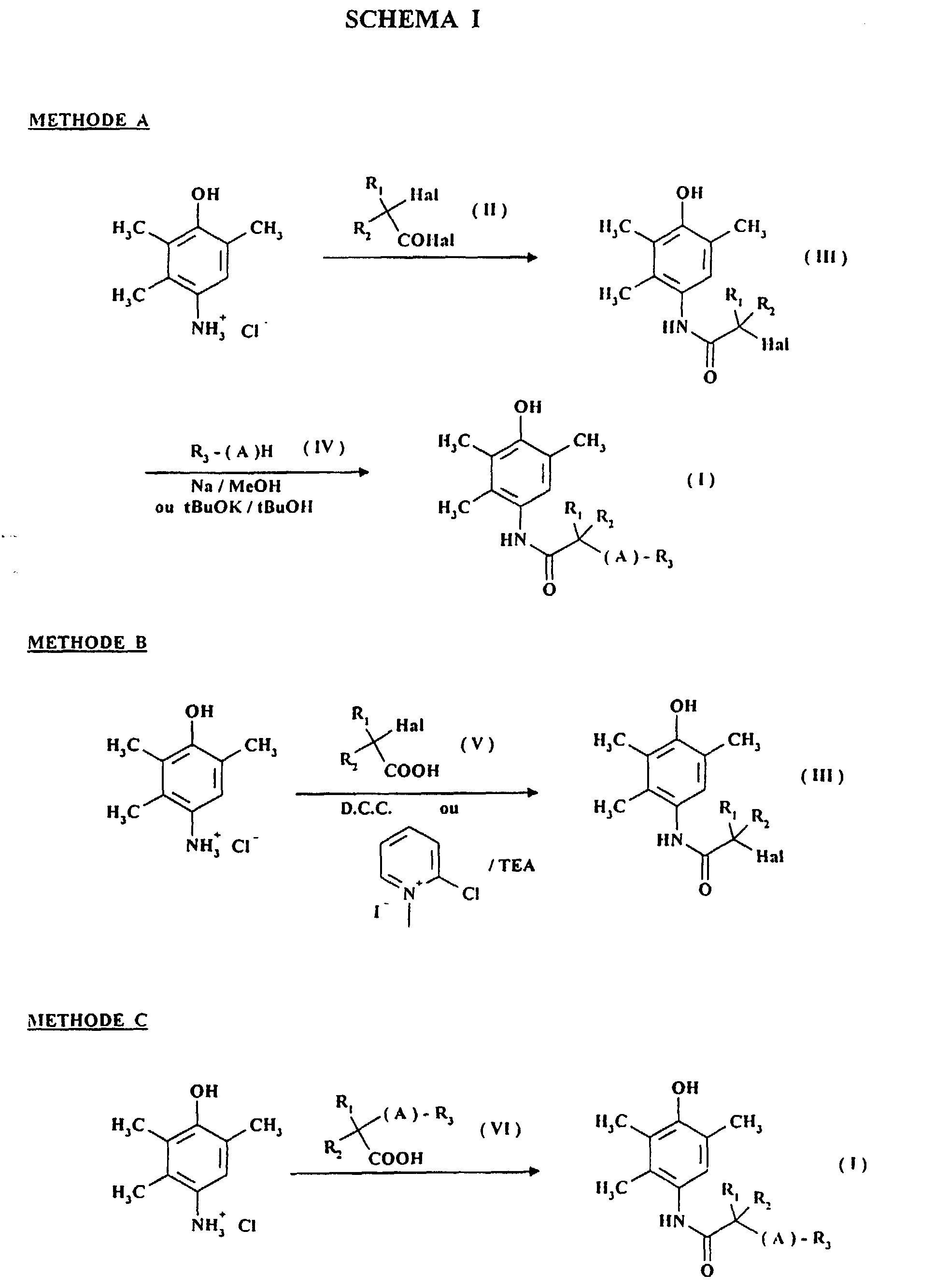

L'invention pourra être mieux comprise à l'aide des exemples non limitatifs qui suivent et qui constituent des modes de réalisation avantageux selon l'invention.

### Exemple 1 :

### (méthode A) 2',3',5'-triméthyl-4'-hydroxy-α-dodécylthio-propionanilide 1.

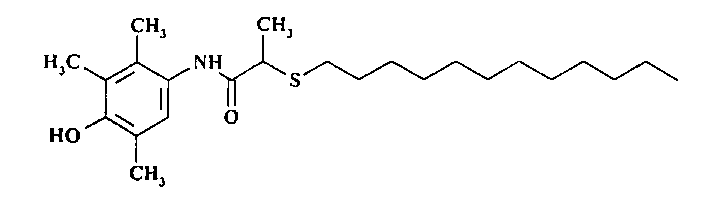

a - 2',3',5'-triméthyl-4'-hydroxy-α-bromo-propionanilide 1a.

A une solution de chlorhydrate de 2,3,6-triméthyl-4-aminophénol (1,87g ; 0,01 mole) dans le diméthylformamide placée sous azote, on ajoute la triéthylamine (3,48 ml ; 0,25 mole) - on additionne ensuite goutte à goutte le chlorure d'α-bromo propionyle (1,32 ml ; 0,0125 mole) et on agite le mélange réactionnel pendant une heure à température ambiante.

Après dilution à l'eau, on extrait à l'acétate d'éthyle. La phase organique est lavée à l'acide chlorhydrique N et à l'eau puis séchée (Mg SO₄) et concentrée à sec sous vide. Le résidu est repris à l'hexane, filtré et séché pour donner le composé 1a (2,10 g).
F = 186°C
CCM : gel de silice 60F254 Merck Rf = 0,61 (AcOEt).

b - 2',3',5'-triméthyl-4'-hydroxy-α-dodécylthio-propionanilide 1.
Le n-dodécanéthiol (2,11 ml ; 0,0088 mole) est dissous dans le méthanol (20 ml) puis additionné de méthylate de sodium (0,47 g ; 0,0088 mole). Après 15 minutes de contact, le composé 1a (2,10 g ; 0,0073 mole) est ajouté et la masse réactionnelle portée à 60°C pendant 2 heures. Le méthanol est ensuite évaporé puis le résidu est extrait à l'acétate d'éthyle.

La phase organique, lavée à l'eau et séchée (MgS0₄) est concentrée à sec sous vide. Le résidu obtenu est purifié par chromatographie flash (élution acétate d'éthyle - hexane 30-70) pour donner 1,24 g de cristaux blancs (1).
F = 123°C
CCM : gel de silice 60F254 Merck Rf= 0,59 (AcOEt - Hexane 50-50).

### Exemple 2 :

### 2',3',5'-triméthyl-4'-hydroxy-α-dodécylthio-acétanilide 2.

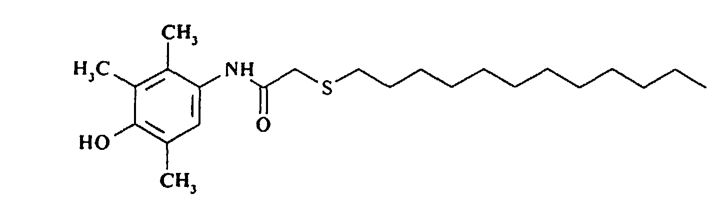

Ce composé est préparé selon le procédé décrit à l'exemple 1 en utilisant le bromure de bromoacétyle. F = 99°C
CCM : gel de Silice 60F254 Merck Rf= 0,51 (AcOEt - Hexane 50-50).

### Exemple 3 :

### 2',3',5'-triméthyl-4'-hydroxy-α-dodécylthio-butyranilide 3.

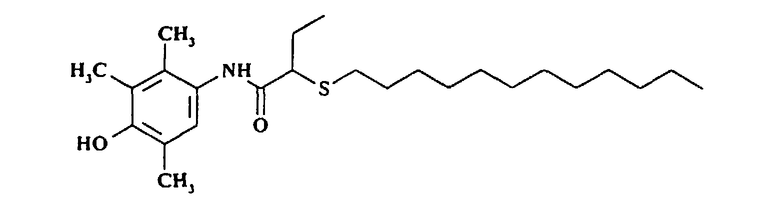

Ce composé est préparé selon le procédé décrit à l'exemple 1 en utilisant le bromure de 2-bromobutyryle. F = 127°C
CCM : gel de silice 60F254 Merck Rf= 0,61 (AcOEt - Hexane 50-50).

### Exemple 4 :

### 2',3',5'-triméthyl-4'-hydroxy-α-dodécylthio-hexananilide 4.

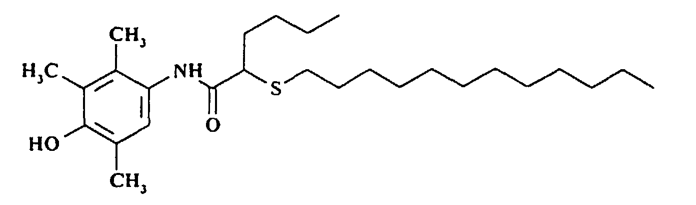

Ce composé est préparé selon le procédé décrit à l'exemple 1 en utilisant le bromure de 2-bromohexanoyle.
F = 80°C
CCM : gel de silice 60F254 Merck Rf= 0,36 (AcOEt - Hexane 30-70).

### Exemple 5 :

### 2',3',5'-triméthyl-4'-hydroxy-α-dodécylthio-isovaléranilide 5.

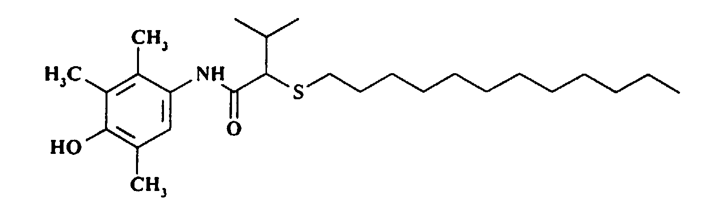

Ce composé est préparé selon le procédé décrit à l'exemple 1 en utilisant le chlorure de 2-bromoisovaleryle.
F = 123°C
CCM : gel de silice 60F254 Merck Rf= 0,30 (AcOEt - Hexane 30-70).

### Exemple 6 :

### 2',3',5'-triméthyl-4'-hydroxy-α-dodécylthio-valéranilide 6.

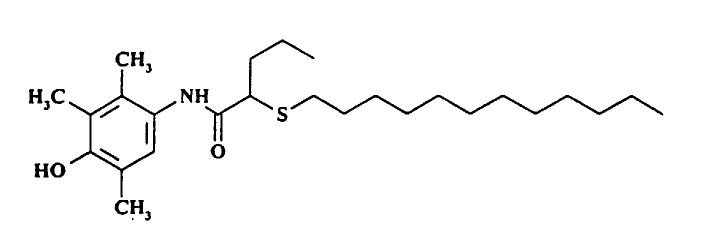

Ce composé est préparé selon le procédé décrit à l'exemple 1 en utilisant le bromure de 2-bromovaléryle.
F = 116°C
CCM : gel de silice 60F254 Merck Rf = 0,39 (AcOEt - Hexane 30-70).

### Exemple 7 :

### (méthode B) 2',3',5'-triméthyl-4-hydroxy-α-dodécylthio-α-phényl acétanilide 7.

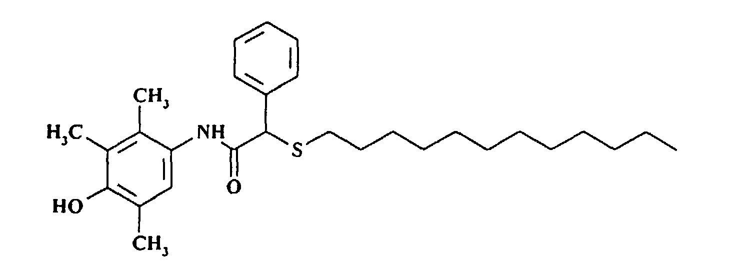

a) -2',3',5'-triméthyl- 4'-hydroxy-α-chloro-α-phényl acétanilide 7.
A une suspension de chlorhydrate de 2,3,6-triméthyl-4-aminophénol (1,27 g ; 0,0067 mole) dans le chlorure de méthylène (35 ml) placée sous azote, on ajoute la triéthylamine (0,94 ml ; 0,0067 mole).

L'acide α-chlorophénylacétique (1,27 g ; 0,0074 mole) et le dicyclohexyl carbodiimide (1,54 g ; 0,0074 mole) sont ensuite ajoutés et le mélange réactionnel agité énergiquement pendant 2 heures à température ambiante.
Après filtration de la dicyclohexyl urée formée, la phase organique est lavée à l'acide chlorhydrique N/10, à l'eau puis à l'eau salée. Après séchage (MgS0₄) et évaporation à sec sous vide, le résidu est repris à l'éther éthylique. Les cristaux formés sont filtrés et séchés pour donner le composé 7a (1,22 g).
F = 199°C
CCM : gel de silice 60F254 Merck Rf= 0.68 (AcOEt - Hexane 50-50).

b) 2',3',5'-triméthyl-4'-hydroxy-α-dodécylthio-α-phényl acétanilide 7.

Le composé est préparé selon la technique décrite à l'exemple 1b, en partant du composé 7a.
F = 129°C
CCM : gel de silice 60F254 Merck Rf= 0,64 (AcOEt - Hexane 50-50).

### Exemple 8:

### (méthode C) 2',3',5'-triméthyl-4'-hydroxy-α-dodécylthio-isobutyranilide 8.

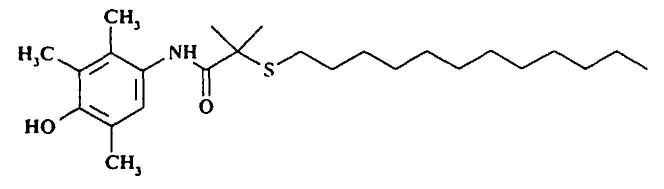

A une suspension d'iodure de 2-chloro-1-méthyl-pyridinium (5,75 g ; 0,022 mole) dans le chloroforme (225 ml), on ajoute successivement le chlorhydrate de 2,3,6- triméthyl-4-amino-phénol (3,52 g ; 0,018 mole), l'acide α-dodécylthio-isobutyrique (5,41 g ; 0,018 mole) et la triéthylamine (9,4 ml ; 0,067 mole) puis chauffe 2 heures à reflux - La masse réactionnelle est refroidie, diluée à l'éther éthylique (350 ml) puis filtrée. Cette phase organique est ensuite lavée à l'acide chlorhydrique N, à l'eau puis à l'eau salée. Après séchage (MgS0₄) et concentration à sec sous vide, le résidu est repris à l'éther isopropylique et filtré pour donner 7,32 g de cristaux blancs du composé 8.
F = 71°C
CCM : gel de silice 60F254 Merck Rf= 0,65 (AcOEt - Hexane 50-50).

### Exemple 9:

### 2',3',5'-triméthyl-4'-hydroxy-α-p. chlorophényl thio-isobutyranilide 9.

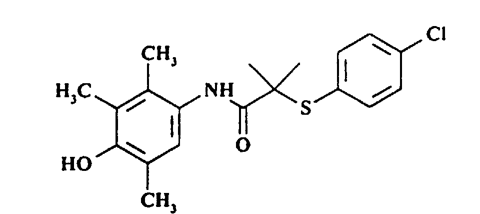

Ce composé est préparé selon le procédé décrit à l'exemple 8, en utilisant l'acide α-p.chlorophénylthioisobutirique.
F = 134°C
CCM : gel de silce 60F254 Merck Rf = 0,54 (AcOEt - Hexane 50-50).

### Exemple 10 :

### 2',3',5'-triméthyl-4'-hydroxy-α-p.chlorophényl sulfinyl isobutyranilide 10.

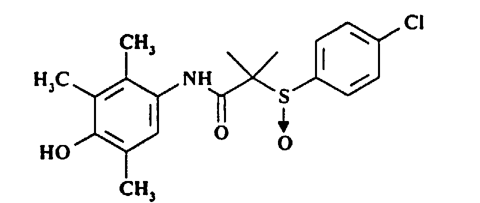

Ce composé est préparé selon le procédé décrit à l'exemple 8 , en utilisant l'acide α-p. chloro phényl sulfinyl isobutyrique.
F = 157 - 158°C
CCM : gel de silice 60F254 Merck Rf = 0,33 (AcOEt - Hexane 50-50).

### Exemple 11 :

### 2',3',5'-triméthyl-4'-hydroxy-α-p. chloro phénoxy isobutyranilide.

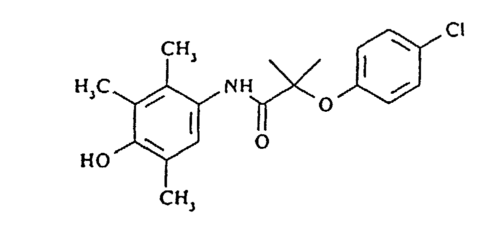

A une solution d'acide clofibrique (2,14 g ; 0,01 mole) et de triéthylamine (1,48 ml ; 0,0105 mole) dans le tétrahydrofurane (25 ml) refroidie à 0°C, on ajoute goutte à goutte le chloroformate d'éthyle (0,96 ml ; 0,01 mole). Après 20 minutes d'agitation, l'anhydride mixte obtenu est additionné lentement à une suspension de chlorhydrate de 2,3,6-triméthyl-4-aminophénol (1,87 g ; 0,01 mole) dans le diméthyl formamide (10 ml) et la triéthylamine (1,48 ml ; 0,0105 mole).

Le mélange réactionnel maintenu sous courant d'azote, est agité 1 heure à 5°C puis 12 heures à température ambiante - puis versé dans l'eau et extrait à l'acétate d'éthyle - La phase organique est lavée à l'eau, à l'eau salée, séchée sur MgS0₄ puis évaporée à sec sous vide. Le résidu est cristallisé dans l'éther éthylique puis recristallisé dans l'acétate d'éthyle pour donner le composé 11.
F = 175°C
CCM : gel de silice 60F254 Merck Rf = 0,30 (AcOEt - Hexane 30-70).

### Exemple 12 :

### 2',3',5'-triméthyl-4'-hydroxy-α-dodécyl sulfinyl isobutyranilide 12.

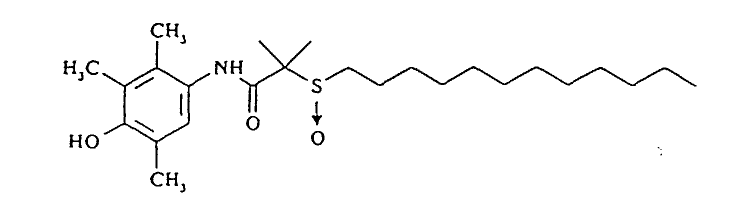

Ce composé est préparé selon le procédé décrit à l'exemple 8 en utilisant l'acide α-dodécyl sulfinylisobutyrique.
F = 73°C
CCM : gel de silice 60F254 Merck Rf= 0,43 (AcOEt - Hexane).

### Exemple 13 :

### 2',3',5'-triméthyl-4'-hydroxy-α-dodécylthio-α-(3,5,diterbutyl-4-hydroxy)-phényl acétanilide 13.

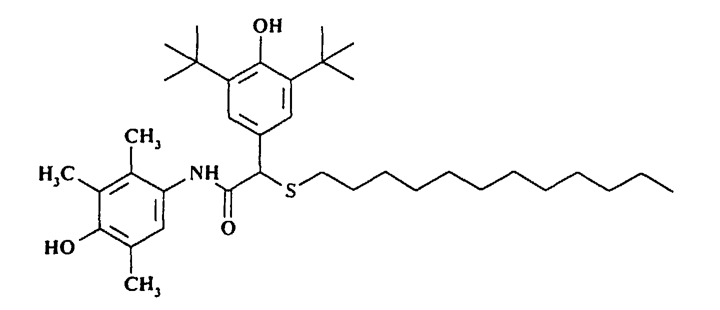

Ce composé est préparé selon la méthode décrite à l'exemple 11 en utilisant l'acide α-dodécylthio-3,5-diterbutyl-4-hydroxy phényl acétique.
F = 150°C
CCM : gel de silice 60F254 Merck Rf= 0,31 (AcOEt - Hexane 30-70).

### Exemple 14 :

### 2',3',5'-triméthyl-4-hydroxy-α-dodécylthio-α-p. méthoxy phényl acétanilide 14.

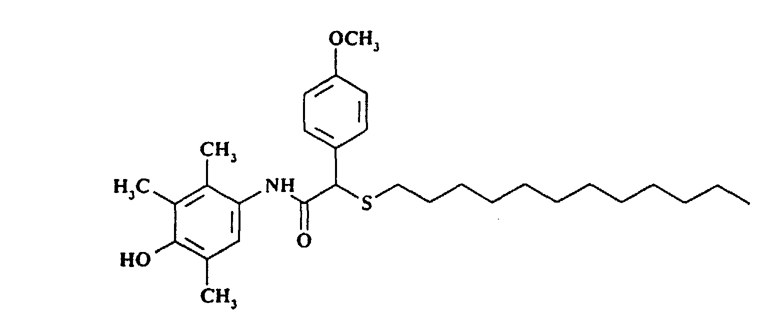

Ce composé est préparé selon la méthode décrite à l'exemple 7a en utilisant l'acide α-dodécyl thio-α-p. méthoxy phényl acétique.
F = 122°C
CCM : gel de silice 60F254 Merck Rf= 0,74 (AcOEt - Hexane 30-70).

### Exemple 15 :

### 2',3',5'-triméthyl-4'-hydroxy-α-dodécylthio-α-naphtyl acétanilide 15.

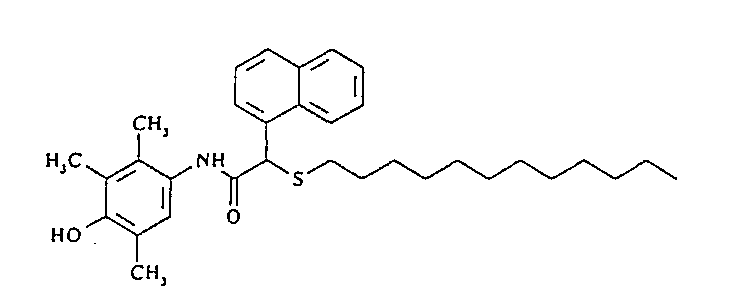

Ce composé est préparé selon la méthode décrite à l'exemple 7a en utilisant l'acide α-dodécylthio-α-naphtyl acétique.
F = 134°C
CCM : gel de silice 60F254 Merck Rf: 0,60 (CH₂Cl₂ - AcOEt 95-5).

### Exemple 16 :

### (+) 2',3',5'-triméthyl-4'-hydroxy-α-dodécylthio-α-phényl acétanilide 16.

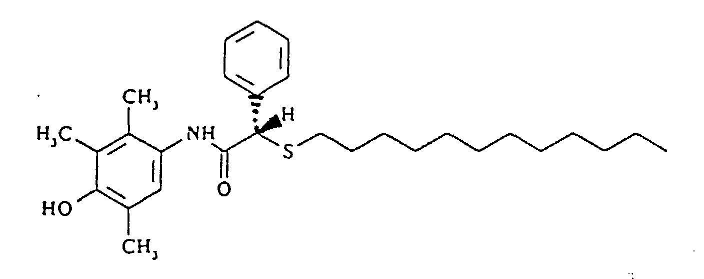

Ce composé est préparé selon la méthode décrite à l'exemple 7a en utilisant l'acide (+)α-dodécylthio-α-phényl acétique.
F = 128°C
CCM : gel de silice 60F254 Merck Rf= 0.64 (AcOEt - Hexane 50-50).
α_{D}²⁵ = + 34,7° (C = 0,5; Ethanol).

Les composés de l'invention ont été soumis à des essais pharmacologiques qui ont montré leur intérêt potentiel dans le traitement de l'hypercholestérolémie et dans le traitement de la maladie athéromateuse.

Les composés ont été étudiés pour leur effet inhibiteur de l'ACAT et hypocholestérolémiant chez le rat d'une part et pour leur effet antioxydant d'autre part.

### 1) Inhibition de l'ACAT :

L'activité inhibitrice de l'ACAT (enzyme acyl CoA : cholestérol O acyl transférase) des composés a été évaluée in vitro en utilisant la technique de H. CHAUTAN et al. (Analytical Biochemistry, 173, 436-439, 1988).

Les activités, exprimées en concentrations inhibitrices 50 % (CI₅₀) obtenues avec certains produits de l'invention sont reportées, à titre d'exemple dans le tableau I suivant :

**Tableau I**

| Composés n° | CI₅₀ (µM) |
|---|---|
| 2 | 0,30 |
| 3 | 0,31 |
| 4 | 0,16 |
| 5 | 0,63 |
| 6 | 0,11 |
| 7 | 0,18 |
| 8 | 0,19 |
| 9 | 1,12 |
| 11 | 1,10 |
| 16 | 0,16 |
| Cl 976 | 1,04 |
| DUP 128 | 0,1 |

CI 976 = N-(2,4,6-triméthoxy)phényl-2,2-diméthyl-dodécanamide.
DUP 128 = N'-(2,4-difluorophényl)-N-[5-[(4,5-diphényl-1H-imidazol-2-yl)thio]pentyl]-N-heptylurée.

### 2) Activité hypocholestérolémiante :

Des rats mâles (160-180 g) sont soumis pendant 4 jours à un régime hypercholestérolémique Altromin C 1061 et traités parallèlement par voie orale par les composés en suspension dans une solution de Tween 80 à 2 % dans de l'eau distillée.

Le 5ème jour, les animaux non à jeun sont anesthésiés à l'éther éthylique, exsanguinés par prélèvement sur EDTA à l'aorte abdominale. Le sang est immédiatement centrifugé et le plasma conservé à 4°C.

Le cholestérol plasmatique est alors dosé par la méthode CHOD-PAP (Boehringer Mannheim Réf. 237574). La dose efficace 50 (DE₅₀) correspond à la dose qui réduit de moitié la concentration en cholestérol plasmatique par rapport aux animaux témoins.

| Composés n° | DE₅₀ (mg/kg) |
|---|---|
| 2 | > 10 |
| 3 | 4 |
| 4 | 0,5 |
| 5 | 1 |
| 6 | 1 |
| 7 | 0,2 |
| 8 | 10 |
| 14 | 1 |
| 16 | 0,15 |
| CI 976 | 8,3 |
| DUP 128 | 1,1 |

### 3) Activité antioxydante :

### a) Péroxydation chimique.

En présence de Fe³⁺ et d'ADP, l'acide dihydroxy fumarique subit une autooxydation qui génère des radicaux libres oxygénés. Ces derniers entraînent la péroxydation des lipides microsomiaux hépatiques.

Cette péroxydation, effectuée sur des microsomes de foie de rat, est mesurée selon la technique à l'acide thiobarbiturique (formation de TBARS) telle que décrite par S.Y.H. TSE et al. (Biochemical Pharmacology, Vol 42, n° 3, 459-464, 1991).

| Composés n° | CI₅₀ (µM) |
|---|---|
| 2 | 5 |
| 3 | > 10 |
| 4 | 0,5 |
| 5 | 5 |
| 6 | 0,3 |
| 7 | 0,6 |
| 8 | 3 |
| CI 976 | > 10 |
| DUP 128 | > 10 |
| Vitamine E | 2,3 |

### b) Oxydation des LDL.

Les LDL humaines (Sigma L 2139) sont oxydées par CuSO₄ 10 µM. Après une période d'incubation de 6 heures, la péroxydation est évaluée par mesure des TBARS par spectrophotométrie à 532 nanomètres.

| Composés n° | CI₅₀ (µM) |
|---|---|
| 4 | 10 |
| 7 | 13 |
| 12 | 3 |
| 16 | 4 |
| CI 976 | 100 |
| DUP 128 | 30 |
| Vitamine E | 10 |

Les composés de l'invention sont des hypocholestérolémiants inhibiteurs d'ACAT et antioxydants qui peuvent être utilisés pour le traitement des maladies telles que l'hypercholestérolémie et l'athérosclérose.

Les compositions pharmaceutiques peuvent être présentées sous la forme appropriée pour l'administration par voie orale, parentérale ou locale, par exemple sous forme de capsules, comprimés, granulés, gélules, solutés liquides, sirops ou suspensions buvables et contenir les excipients appropriés.

La posologie quotidienne peut aller de 10 à 3000 mg.

## Revendications

1. Dérivés d'anilides caractérisés en ce qu'ils répondent à la formule générale I 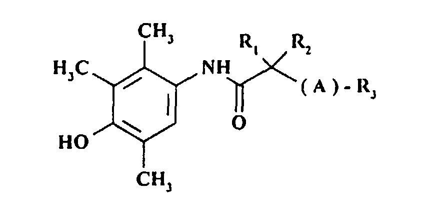 dans laquelle :
. R₁ et R₂ identiques ou différents représentent indépendamment l'un de l'autre :
. l'hydrogène
. un radical alcoyle linéaire ou ramifié en C₁ - C₆
. un groupement aromatique tel que phényle, naphtyle ou pyridyle éventuellement substitué par un ou plusieurs groupements alcoyle en C₁ - C₄, alcoxy en C₁ - C₄, hydroxyle ou halogéno;
. R3 représente une chaîne alcoyle linéaire ou ramifiée en C6 - C15 ou un groupement phényle éventuellement substitué par un ou plusieurs groupements alcoyle en C₁ - C₄, alcoxy en C₁ - C₄, hydroxyle ou halogéno;
. A représente un atome d'oxygène ou de soufre ou le groupement sulfoxy;
sous forme de leurs différents stéréoisomères ou énantiomères, ainsi que leurs mélanges, pour les composés présentant un ou plusieurs autres asymétriques;

2. Composés répondant à la formule générale I, selon la revendication 1, sélectionnés parmi le groupe suivant :
- 2',3',5'-triméthyl-4'-hydroxy-α-dodécylthio-propionanilide.
- 2',3',5'-triméthyl-4'-hydroxy-α-dodécylthio-acétanilide.
- 2',3',5'-triméthyl-4'-hydroxy-α-dodécylthio-butyranilide.
- 2',3',5'-triméthyl-4'-hydroxy-α-dodécylthio-hexananilide.
- 2',3',5'-triméthyl-4'-hydroxy-α-dodécylthio-isovaléranilide.
- 2',3',5'-triméthyl-4'-hydroxy-α-dodécylthio-valéranilide.
- 2',3',5'-triméthyl-4'-hydroxy-α-dodécylthio-α-phényl acétanilide.
- 2',3',5'-triméthyl-4'-hydroxy-α-dodécylthio-isobutyranilide.
- 2',3',5'-triméthyl-4'-hydroxy-α-p. chlorophénylthio-isobutyranilide.
- 2',3',5'-triméthyl-4'-hydroxy-α-p. chlorophényl sulfinyl isobutyranilide.
- 2',3',5'-triméthyl-4'-hydroxy-α-p. chlorophénoxy isobutyranilide.
- 2',3',5'-triméthyl-4'-hydroxy-α-dodécyl sulfinyl bobutyranilide.
- 2',3',5'-triméthyl-4'-hydroxy-α-dodécylthio-α-(3,5-diterbutyl-4-hydroxy)-phényl acétanilide.
- 2',3',5'-triméthyl-4'-hydroxy-α-dodécylthio-α-p.méthoxy phényl acétanilide.
- 2',3',5'-triméthyl-4'-hydroxy-α-dodécylthio-α-naphtyl acétanilide.
-(+)2',3',5'-triméthyl-4'-hydroxy-α-dodécylthio-α-phényl acétanilide;

3. Procédé de préparation des composés selon l'une des revendicatons 1 ou 2, caractérisé en ce que :
a) - dans une première étape, on traite le chlorhydrate de 2,3,6-triméthyl-4-aminophénol par un α-halogéno-halogénure d'acyle en présence d'une base telle que la triéthylamine pour fournir l'intermédiaire III 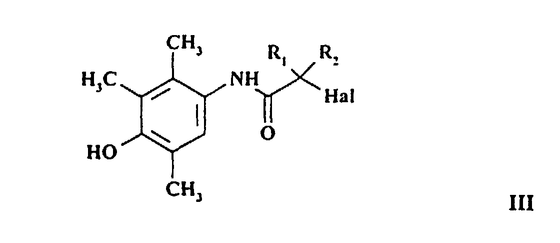 dans lequel R₁, R₂ sont tels que définis dans la revendication 1 et Hal représente un atome de chlore ou de brome;
b) - dans une deuxième étape on traite l'intermédiaire III par un dérivé R₃ (A)H, dans lequel R₃ et A sont tels que définis dans la revendication 1, dans un milieu sodium-méthanol ou tertiobutylate de potassium - tertiobutanol;

4. Procédé de préparation des composés selon la revendication 3 caractérisé en ce que l'intermédiaire III peut être obtenu par action d'un α halogéno acide en présence d'un activateur tel que le dicyclohexylcarbodiimide ou l'iodure de 2-chloro-1-méthyl-pyridinium et d'une base telle que la triéthylamine sur le chlorhydrate de 2,3,6-triméthyl-4-aminophénol;

5. Procédé de préparation des composés selon les revendications 1 et 2 caractérisé en ce que l'on traite le chlorhydrate de 2,3,6-triméthyl-4-amino phénol par un dérivé VI 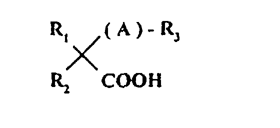 dans lequel R₁, R₂, R₃ et A sont tels que définis dans la revendication 1, en activant par le chloroformate d'éthyle ou par le dicyclohexylcarbodiimide ou l'iodure de 2-chloro-1-méthylpyridinium en présence d'une base telle que la triéthylamine;

6. A titre de médicaments, les composés de formule générale I selon les revendications 1 ou 2, en particulier à titre de médicaments utilisés pour le traitement des maladies telles que l'hypercholestérolémie ou l'athérosclérose;

7. Compositions pharmaceutiques, caractérisées par le fait qu'elles contiennent, outre un support pharmaceutiquement acceptable, au moins un composé de formule générale I selon l'une quelconque des revendications 1 et 2;

8. Utilisation de composés de formule générale I selon l'une quelconque des revendications 1 ou 2, pour la fabrication de médicaments destinés au traitement de maladies telles que l'hypercholestérolémie ou l'athérosclérose.

## Patentansprüche

1. Anilid-Derivate, dadurch charakterisiert, daß sie der allgemeinen Formel I entsprechen 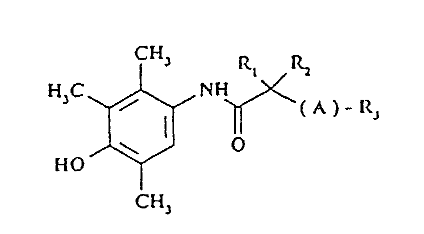 wobei:
R₁ und R₂, identisch oder verschieden, unabhängig voneinander darstellen:
- Wasserstoff
- ein lineares oder verzweigtes (C₁-C₆)Alkylradikal
- eine aromatische Gruppe wie Phenyl, Naphthyl oder Pyridyl, gegebenenfalls substituiert durch eine oder mehrere (C₁-C₄)Alkyl-, (C₁-C₄)Alkoxy-, Hydroxyl- oder Halogengruppen;
- R₃ eine lineare oder verzweigte (C₆-C₁₅)Alkylkette oder eine Phenylgruppe darstellt, gegebenenfalls substituiert durch eine oder mehrere (C₁-C₄)Alkyl-, (C₁-C₄)Alkoxy-, Hydroxyl- oder Halogengruppen;
- A ein Sauerstoff- oder Schwefelatom oder eine Sulfoxygruppe darstellt;
in Form ihrer verschiedenen Stereoisomere oder Enantiomere genauso wie ihre Gemische für die Verbindungen, die eine oder mehrere weitere asymmetrische Zentren aufweisen.

2. Der allgemeinen Formel I entsprechende Verbindungen nach Anspruch 1, ausgewählt aus der folgenden Gruppe:
- 2',3',5'-Trimethyl-4'-hydroxy-α-docecylthio-propionanilid,
- 2',3',5'-Trimethyl-4'-hydroxy-α-docecylthio-acetanilid,
- 2',3',5'-Trimethyl-4'-hydroxy-α-docecylthio-butyranilid,
- 2',3',5'-Trimethyl-4'-hydroxy-α-docecylthio-hexananilid,
- 2',3',5'-Trimethyl-4'-hydroxy-α-docecylthio-isovaleranilid,
- 2',3',5'-Trimethyl-4'-hydroxy-α-docecylthio-valeranilid,
- 2',3',5'-Trimethyl-4'-hydroxy-α-docecylthio-α-phenyl-acetanilid,
- 2',3',5'-Trimethyl-4'-hydroxy-α-docecylthio-isobutyranilid,
- 2',3',5'-Trimethyl-4'-hydroxy-α-p-chlorophenylthio-isobutyranilid,
- 2',3',5'-Trimethyl-4'-hydroxy-α-p-chlorophenylsulfinylisobutyranilid,
- 2',3',5'-Trimethyl-4'-hydroxy-α-p-chlorophenoxyisobutyranilid,
- 2',3',5'-Trimethyl-4'-hydroxy-α-dodecylsulfinylbobutyranilid,
- 2',3',5'-Trimethyl-4'-hydroxy-α-dodecylthio-α-(3,5-di-tert-butyl-4-hydroxy)phenylacetanilid,
- 2',3',5'-Trimethyl-4'-hydroxy-α-dodecylthio-α-p-methoxyphenylacetanilid,
- 2',3',5'-Trimethyl-4'-hydroxy-α-dodecylthio-α-naphthylacetanilid.
- (+)2',3',5'-Trimethyl-4'-hydroxy-α-dodecylthio-α-phenylacetanilid.

3. Verfahren zur Herstellung von Verbindungen nach einem der Ansprüche 1 oder 2, dadurch charakterisiert, daß:
a) man in einem ersten Schritt das Hydrochlorid von 2,3,6-Trimethyl-4-aminophenol mit einem α-Halogeno-acylchlorid in Gegenwart einer Base wie Triethylamin zur Bildung des Zwischenprodukts III 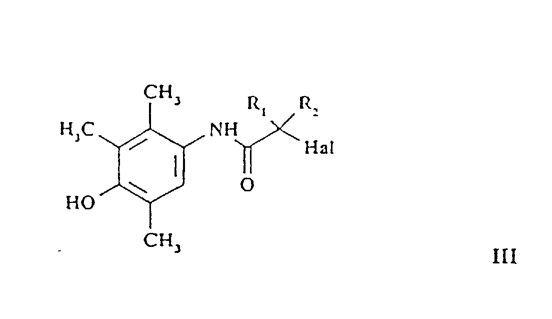 behandelt, wobei R₁ und R₂ so wie in Anspruch 1 definiert sind und Hal ein Chlor- oder Bromatom darstellt;
b) man in einem zweiten Schritt das Zwischenprodukt III mit einem Derivat R₃ (A)H behandelt, wobei R₃ und A wie in Anspruch 1 definiert sind, in einem Medium Natrium-Methanol oder Kalium-tert-butylat-tert-Butanol.

4. Verfahren zur Herstellung von Verbindungen nach Anspruch 3, dadurch charakterisiert, daß das Zwischenprodukt III durch Einwirkung eines α-Halogen-Säure in Gegenwart eines Aktivators wie Dicyclohexylcarbodiimid oder 2-Chloro-1-methylpyridiniumiodid und einer Base wie Triethylamin auf das Hydrochlorid von 2,3,6-Trimethyl-4-aminophenol erhalten werden kann.

5. Verfahren zur Herstellung von Verbindungen nach den Ansprüchen 1 und 2, dadurch charakterisiert, daß man das Hydrochlorid von 2,3,6-Trimethyl-4-aminophenol mit einem Derivat VI 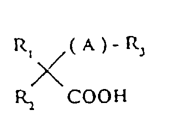 behandelt, wobei R₁, R₂, R₃ und A wie in Anspruch 1 definiert sind, indem durch Ethylchloroformiat oder durch Dicyclohexylearbodiimid oder 2-Chloro-1-methylpyridiniumiodid in Gegenwart einer Base wie Triethylamin aktiviert wird.

6. Verbindungen der allgemeinen Formel I nach den Ansprüchen 1 oder 2 als Medikamente; insbesondere als Medikamente, die für die Behandlung von Erkrankungen wie Hypercholesterolämie oder Atherosklerose verwendet werden.

7. Pharmazeutische Zusammensetzungen, dadurch charakterisiert, daß sie außer einem pharmazeutisch verträglichen Träger mindestens eine Verbindung der allgemeinen Formel I nach einem der Ansprüche 1 und 2 enthalten.

8. Verwendung von Verbindungen der allgemeinen Formel I nach einem der Ansprüche 1 oder 2 zur Herstellung von Medikamenten, die für die Behandlung von Erkrankungen wie Hypercholesterolämie oder Artherosklerose bestimmt sind.

## Claims

1. Anilide derivatives, characterized in that they correspond to the general formula I 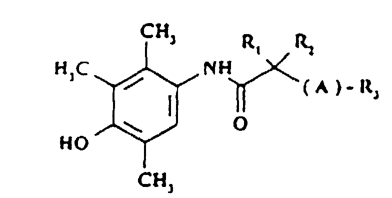 in which:
• R₁ and R₂, which are identical or different, represent, independently of one another:
• hydrogen
• a linear or branched C₁-C₆ alkyl radical
• an aromatic group, such as phenyl, naphthyl or pyridyl, optionally substituted by one or more C₁-C₄ alkyl, C₁-C₄ alkoxy, hydroxyl or halo groups;
• R₃ represents a linear or branched C₆-C₁₅ alkyl chain or a phenyl group optionally substituted by one or more C₁-C₄ alkyl, C₁-C₄ alkoxy, hydroxyl or halo groups;
• A represents an oxygen or sulphur atom or the sulphoxy group;
in the form of their various stereoisomers or enantiomers, and their mixtures, for the compounds exhibiting one or more asymmetric centres.

2. Compounds corresponding to the general formula I according to Claim 1, selected from the following group:
- 2',3',5'-trimethyl-4'-hydroxy-α-(dodecylthio)propionanilide.
- 2',3',5'-trimethyl-4'-hydroxy-α-(dodecylthio)acetanilide.
- 2',3',5'-trimethyl-4'-hydroxy-α-(dodecylthio)butyranilide.
- 2',3',5'-trimethyl-4'-hydroxy-α-(dodecylthio)hexananilide.
- 2',3',5'-trimethyl-4'-hydroxy-α-(dodecylthio)isovaleranilide.
- 2',3',5'-trimethyl-4'-hydroxy-α-(dodecylthio)valeranilide.
- 2',3',5'-trimethyl-4'-hydroxy-α-dodecylthio-α-phenylacetanilide.
- 2',3',5'-trimethyl-4'-hydroxy-α-(dodecylthio)isobutyranilide.
- 2',3',5'-trimethyl-4'-hydroxy-α-(p-chlorophenylthio)isobutyranilide.
- 2',3',5'-trimethyl-4'-hydroxy-α-(p-chlorophenylsulphinyl)isobutyranilide.
- 2',3',5'-trimethyl-4'-hydroxy-α-(p-chlorophenoxy)isobutyranilide.
- 2',3',5'-trimethyl-4'-hydroxy-α-(dodecylsulphinyl)bobutyranilide.
- 2',3',5'-trimethyl-4'-hydroxy-α-dodecylthioα-(3,5-di-tert-butyl-4-hydroxyphenyl)acetanilide.
- 2',3',5'-trimethyl-4'-hydroxy-α-dodecylthioα-(p-methoxyphenyl)acetanilide.
- 2',3',5'-trimethyl-4'-hydroxy-α-dodecylthioα-naphthylacetanilide.
- (+)-2',3',5'-trimethyl-4'-hydroxy-α-dodecylthioα-phenylacetanilide.

3. Process for the preparation of the compounds according to either of Claims 1 and 2, characterized in that:
a) - in a first stage, 2,3,6-trimethyl-4-aminophenol hydrochloride is treated with an α-haloacyl halide in the presence of a base, such as triethylamine, in order to provide the intermediate III 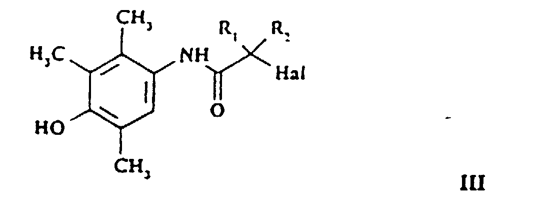 in which R₁ and R₂ are as defined in Claim 1 and Hal represents a chlorine or bromine atom;
b) - in a second stage, the intermediate III is treated with a derivative R₃(A)H, in which R₃ and A are as defined in Claim 1, in a sodium/methanol or potassium tert-butoxide/tert-butanol medium.

4. Process for the preparation of the compounds according to Claim 3, characterized in that the intermediate III can be obtained by reaction of an α-halo acid with 2,3,6-trimethyl-4-aminophenol hydrochloride in the presence of an activator, such as dicyclohexylcarbodiimide or 2-chloro-1-methylpyridinium iodide, and of a base, such as triethylamine.

5. Process for the preparation of the compounds according to Claims 1 and 2, characterized in that 2,3,6-trimethyl-4-aminophenol hydrochloride is treated with a derivative VI 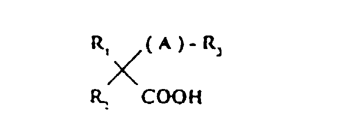 in which R₁, R₂, R₃ and A are as defined in Claim 1, activation being carried out with ethyl chloroformate or with dicyclohexylcarbodiimide or 2-chloro-1-methylpyridinium iodide in the presence of a base, such as triethylamine.

6. As medicaments, the compounds of general formula I according to Claims 1 and 2, in particular as medicaments used in the treatment of diseases such as hypercholesterolaemia or atherosclerosis.

7. Pharmaceutical compositions, characterized in that they contain, in addition to a pharmaceutically acceptable vehicle, at least one compound of general formula I according to either one of Claims 1 and 2.

8. Use of compounds of general formula I according to either one of Claims 1 and 2 in the manufacture of medicaments intended for the treatment of diseases such as hypercholesterolaemia or atherosclerosis.
